# EUROPEAN PATENT APPLICATION

(11) **EP 1 359 150 A2**
(43) Date of publication of application: **05.11.2003**
(21) Application number: 03016679.7
(22) Date of filing: 09.02.1999
(51) Int. Cl.: C07D 487/04, C07C 69/738, C07D 307/16, C07D 211/70, C07D 333/24, A01N 43/90

(54) **A process and intermediates for the preparation of fungicidal 7-alkyl-triazolopyrimidines**

(30) Priority: 11.02.1998 US 22288; 03.02.1999 US 243851
(62) Divisional of application: 99905905.8
(71) Applicant: BASF AKTIENGESELLSCHAFT, 67056 Ludwigshafen (DE)
(72) Inventor: Pfrengle, Waldemar, 55444 Siebersbach (DE); Pees, Klaus-Juergen, 55129 Mainz (DE); Albert, Guido, 55546 Hackenheim (DE)

(57) **Abstract**

A process and intermediates for the preparation of triazolopyrimidine compounds of formula I 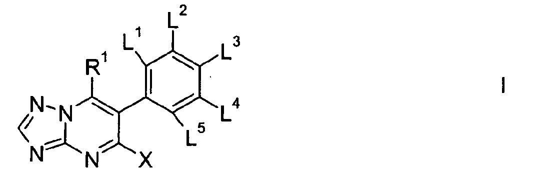 wherein R¹, X, L¹, L², L³, L⁴ and L⁵ are as defined in the description, which comprises reacting alkyl 2-aryl-3-alkyl-3-oxopropionates of formula IV 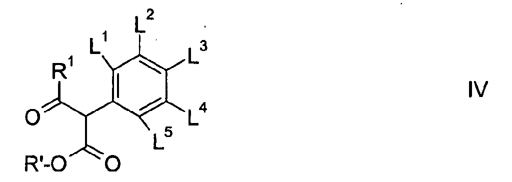 wherein R¹ and L¹, L², L³, L⁴ and L⁵ are as defined for formula I and R' represents an optionally substituted alkyl group,
a) with 2-amino-[1,3,4]-triazole, and treating the resulting 5-hydroxytriazolopyrimidines of formula I, wherein X represents a hydroxy group, with a halogenating agent, and
b) optionally treating the resulting 5-hydroxytriazolopyrimidines with an alcohol or a thioalkohol in the presence of a base, or with a metal amide, a metal alkyl amide or a metal dialkylamide, or a metal cyanide.

## Description

This invention relates to a process and intermediates for the preparation of certain triazolopyrimidine compounds.

EP-A-0 071 792 discloses compounds of the general formula 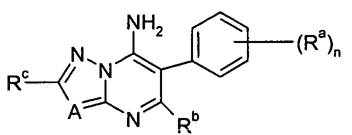 in which R^{b} represents alkyl, halogen, alkoxy, cyano, cycloalkyl, aryl, aryloxy, arylthio, aralkyl, arylalkyl, arylalkyloxy or arylalkylthio each optionally substituted by halogen or alkoxy; or (R^{a})ₙ represents a benzene, indane or tetrahydronaphthalene ring fused with the phenyl ring, aromatic moieties in the above groups being optionally substituted by alkyl, alkoxy, halogen or cyano; n is 1 or 2; R^{b} and R^{c} are each hydrogen, alkyl or aryl, A represents a nitrogen atom or a CR^{d} group, and R^{d} is as R^{b} but can also be halogen, cyano or alkoxycarbonyl or, together with R^{b}, can form an alkylene chain containing up to two double bonds. The compounds are said to be active against various phytopathogenic fungi, especially those of the phycomycete class. However, evidence of fungicidal activity is only provided for these compounds against Plasmopara viticola, a member of the oomycete class of fungi.

U.S. Patent 5,593,996 discloses compounds of the general formula 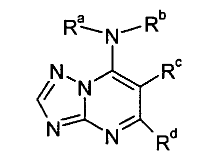 in which R^{a} represents an optionally substituted alkyl, alkenyl, alkadienyl, cycloalkyl, bicycloalkyl or heterocyclyl group; R^{b} represents a hydrogen atom or an alkyl group; or R^{a} and R^{b} together with the interjacent nitrogen atom represent an optionally substituted heterocyclic ring; R^{c} represents an optionally substituted phenyl or naphthyl group; and R^{d} represents a halogen atom or a group -NR^{e}R^{f} where R^{e} represents a hydrogen atom or an amino, alkyl, cycloalkyl or bicycloalkyl group and R^{f} represents a hydrogen atom or an alkyl group.

Makisumi et al., Chem. Pharm Bull. 12 (2) 204-212, (1964) describe the preparation of 5,6,7-trimethyl-s-triazolo[1,5-a]pyrimidine. However, there is no disclosure of any fungicidal activity.

The broad generic formula of U.S. Patent 4,863,843 suggests the use hydroxysubstituted triazolopyrimidines as components of photographic silver halide emulsions. However, there is no single 7-alkyl-6-aryl-5-hydroxytriazolopyrimidine disclosed. Moreover, there is no disclosure of fungicidal properties.

The present invention provides a process and intermediates for the preparation of compounds of formula I 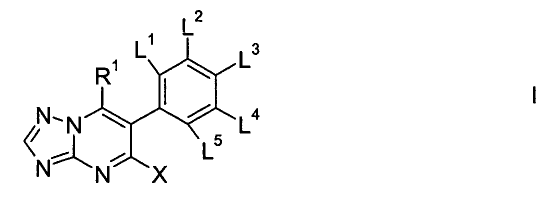 wherein
R¹ represents an optionally substituted alkyl, alkenyl, alkynyl, alkadienyl, or aryl group, or an optionally substituted cycloalkyl or cycloalkenyl group, in which one CH₂ group may also be replaced by O, S or NR², in which R² represents a hydrogen atom or an alkyl group;
X represents a hydrogen or halogen atom, or a hydroxy, alkoxy, aryloxy, aralkyloxy, haloalkoxy, alkylthio, cyano, amino, alkylamino or dialkylamino group;
L¹, L², L³, L⁴ and L⁵ each independently represent a hydrogen or halogen atom or an optionally substituted alkyl or alkoxy group or a nitro or cyano group.

These new compounds show an excellent selective fungicidal activity in various crops.

In general terms, unless otherwise stated, as used herein the term "halogen atom" may denote a bromine, iodine, chlorine or fluorine atom, and is especially a bromine, chlorine or fluorine atom. Optionally substituted moieties may be unsubstituted or have from one up to the maximal possible number of substituents. Typically, 0 to 2 substituents are present.

In general terms, unless otherwise stated herein, the terms "alkyl", "alkenyl", "alkenyl", "alkadienyl" as used herein with respect to a radical or moiety refer to a straight or branched chain radical or moiety. As a rule, such radicals have up to 10, in particular up to 6 carbon atoms. Preferably an alkyl moiety has from 1 to 10 carbon atoms, preferably from 2 to 6 carbon atoms. A preferred alkyl moiety is an ethyl or especially a methyl, group. Preferably, an alkenyl moiety has from 2 to 6 carbon atoms.

In general terms, unless otherwise stated herein, the term "aryl", as used herein with respect to a radical or moiety refers to an aryl group having 6, 10 or 14 carbon atoms, preferably 6 or 10 carbon atoms, in particular, phenyl, being optionally substituted by one or more halogen atoms, nitro, cyano, alkyl, preferably C₁₋₆ alkyl, alkoxy, preferably C₁₋₆ alkoxy, haloalkyl, preferably C₁₋₆ haloalkyl, haloalkoxy, preferably C₁₋₆ haloalkoxy groups.

In general terms, unless otherwise stated herein, the terms "cycloalkyl" or "cycloalkenyl", as used herein with respect to a radical or moiety refer to a cycloalkyl group having 3 to 8 carbon atoms or a cycloalkenyl group having 5 to 8 carbon atoms, preferably 5 to 7 carbon atoms, in particular cyclopentyl, cyclohexyl or cyclohexenyl being optionally substituted by one or more halogen atoms, nitro, cyano, alkyl, preferably C₁₋₆ alkyl, alkoxy, preferably C₁₋₆ alkoxy.

In general terms, unless otherwise stated herein, the term "cycloalkyl or cycloalkenyl, in which one CH₂ group is replaced by O, S or NR²," as used herein with respect to a radical or moiety, refers to a saturated or unsaturated heterocyclyl group having 5 or 6 ring atoms selected from carbon, oxygen, sulfur and nitrogen, one of which being oxygen, sulfur or nitrogen being optionally substituted by one or more halogen atoms, nitro, cyano, alkyl, preferably C₁₋₆ alkyl, alkoxy, preferably C₁₋₆ alkoxy, preferably 2,3-dehydropiperid-3-yl, tetrahydropyranyl, tetrahydrofuranyl or tetrahydrothienyl, in particular N-methyl-2,3-dehydropiperid-3-yl.

Preferred compounds of this are those compounds of the general formula I in which any alkyl part of the groups R¹, R² or X which may be straight chained or branched, contains 1 to 10 carbon atoms, preferably, 2 to 9 carbon atoms, more preferably, 3 to 6 carbon atoms, any alkenyl, alkynyl or alkadienyl part of the substituents R¹ contains 2 to 10 carbon atoms, preferably, 3 to 9 carbon atoms, more preferably, 4 to 6 carbon atoms, any cycloalkyl part of the substituents R¹ contains from 3 to 10 carbon atoms, preferably, from 3 to 8 carbon atoms, more preferably, from 3 to 6 carbon atoms, and any aryl part of the substituents R¹ contains 6, 10 or 14 carbon atoms, preferably, 6 or 10 carbon atoms, and in which each optionally substituted group independently is substituted by one or more halogen atoms or nitro, cyano, alkyl, preferably, C₁₋₆ alkyl, cycloalkyl, preferably, C₃₋₆ cycloalkyl, cycloalkenyl, preferably, C₃₋₆ cycloalkenyl, haloalkyl, preferably C₁₋₆ haloalkyl, halocycloalkyl, preferably C₃₋₆ halocycloalkyl, alkoxy, preferably C₁₋₆ alkoxy, alkanoyloxy, preferably C₁₋₆ alkanoyloxy, haloalkoxy, preferably C₁₋₆ haloalkoxy, alkylthio, preferably C₁₋₆ alkylthio, phenyl, halo-, dihalo- or trihalophenyl or pyridyl groups. Any alkyl, alkenyl or alkynyl group may be linear or branched. A halogen atom suitably denotes a fluorine, chlorine or iodine atom.

Especially preferred compound of this are compounds of the general formula I in which R¹ represents a C₁₋₁₀ alkyl, C₁₋₁₀ haloalkyl, in particular, a fluorinated C₁₋₁₀ alkyl group, C₃₋₆ cycloalkyl, C₃₋₆ cycloalkyl-C₁₋₆ alkyl, C₁₋₆ alkyl-C₃₋₈ cycloalkyl, in particular, a methylcyclohexyl group, halo-C₃₋₆ cycloalkyl, in particular a fluorocyclohexyl, most preferably a 3- or-4-fluorocyclohexyl group, C₅₋₈ cycloalkenyl, C₁₋₁₀ alkoxy-C₁₋₆ alkyl, a phenyl, a mono- or di-C₁₋₆ alkyl-phenyl group, a phenyl-C₁₋₁₀ alkyl, or a mono- or di-C₁₋₆ alkyl-phenyl-C₁₋₁₀ alkyl group, in particular, a benzyl group.

Preferably at least one of the substituents L¹ through L⁵, in particular L¹ and/or L⁵, is different from hydrogen. L¹ is preferably a fluorine or chlorine atom or a methyl, methoxy or trifluoromethoxy group. The other substituents are preferably selected from hydrogen or fluorine.

Also, particularly preferred are compounds of formula I, in which the phenyl group of formula 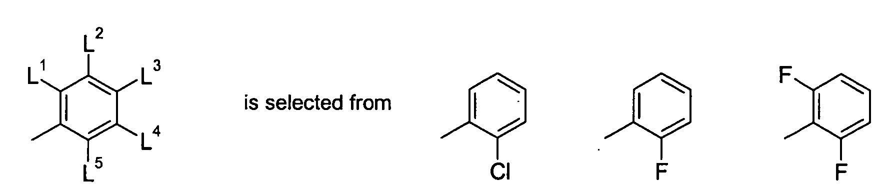 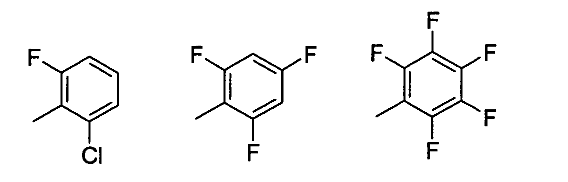 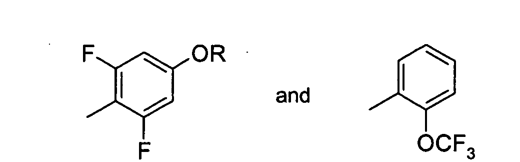 wherein R represents an alkyl group.

Most preferred are the 2-chloro-6-fluorophenyl, the 2,4,6-trifluorophenyl and the 2,6-difluoro-4-methoxyphenyl groups.

Also preferred are compounds of the general formula I in which X represents a halogen atom, in particular, a chlorine or iodine atom, a C₁₋₁₀ alkoxy, in particular, a methoxy or ethoxy group, a C₁₋₁₀ haloalkoxy, in particular a fluorinated C₁₋₁₀ alkoxy group, most preferably, a fluorinated methoxy or ethoxy group, a phenoxy, a mono- or di-C₁₋₆ alkyl-phenoxy group, a phenyl-C₁₋₁₀ alkoxy, or a mono- or di-C₁₋₆ alkylphenyl-C₁₋₁₀ alkoxy group, in particular, a benzyloxy group.

Included in the scope of the present invention are (R) and (S) isomers of compounds of general formula I having a chiral center and the racemates thereof, and salts, N-oxides and acid addition compounds.

The compounds according to general formula I are oils, gums, semi-solids or crystalline solid materials. They are superior by virtue of their valuable fungicidal properties, in particular, their fungitoxicity against a broad range of phythopathogenic fungi. For example, they can be used in agriculture or related fields for the control of phytopathogenic fungi such as *Alternaria solani, Botrytis cinerea, Cercospora beticola, Cladosporium herbarum, Corticium rolfsii, Erysiphe graminis, Helminthosporium tritici repentis, Leptosphaeria nodorum, Micronectriella nivalis, Monilinia fructigena, Mycosphaerella ligulicola, Mycosphaerella pinodes, Phytophthora infestans, Pyricularia grisea f sp. oryzae, Rhizoctonia solani, Monographeila nivalis Sclerotinia sclerotiorum, Uncinula necator* and *Venturia inaequalis,* in particular for the control of, *Alternaria solani Botrytis cinerea* and *Venturia inaequalis.* The compounds of general formula I according to the invention possess a high fungicidal activity within a wide concentration range and may be used in agriculture without any difficulties.

Moreover, the compounds according to the invention show enhanced residual control of fungi compared with conventional fungicides.

Good results in terms of control of phythopathogenic fungi are obtained with a compound as defined in formula I wherein:
X represents a halogen atom, an alkoxy or haloalkoxy group, in particular a chlorine or iodine atom or a methoxy, ethoxy, fluoromethoxy or 2,2,2-difluorethoxy group;
R¹ represents preferably straight chained or branched C₁-C₈-alkyl, in particular n-propyl, iso-propyl, 1- or 2-methylpropyl, n-butyl, n-pentyl or n-hexyl, C₃₋₇-cycloalkyl being optionally substituted by a fluorine atom, a C₁-C₈-alkyl group or a C₂-C₈ alkanoyloxy group, in particular cyclopentyl, cyclohexyl, 4-methylcyclohexyl, 4-acetoxy-cyclohexyl or 3- or 4-fluorocyclohexyl, straight chained or branched C₁-C₆-haloalkyl, in particular 3,3,3-trifluoropropyl, or phenyl being optionally substituted by at least one halogen atom or at least one C₁-C₆-alkyl or C₁-C₆alkoxy group.

Particularly preferred are the compounds of formula lA. 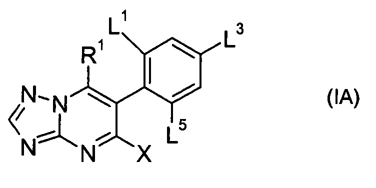 wherein
R¹ is as herein above defined, X represents a chlorine or iodine atom, or a methoxy or ethoxy group, and L¹, L² and L³ each independently represent a hydrogen, fluorine or chlorine atom, or a methoxy, methyl, or trifluoromethoxy group, at least one of which is other than hydrogen.

Especially good results in terms of control of phytopathogenic fungi are obtained by using, for example, the following compounds of formulal:
5-chloro-6-phenyl-7-butyl-[1,2,4]triazolo[1,5-a]pyrimidine, 5-chloro-6-(2-chloro-6-fluorophenyl)-7-butyl-[1,2,4]triazolo[1,5-a]pyrimidine, 5-chloro-6-(2-chloro-6-fluorophenyl)-7-hexyl-[1,2,4]triazolo[1,5-a]pyrimidine, 5-chloro-7-butyl-6-(2,4,6-trifluorophenyl)-[1,2,4]triazolo[1,5-a]pyrimidine, 5-chloro-7-butyl-6-(2-methylphenyl)-[1,2,4]triazolo[1,5-a]pyrimidine, 5-chloro-7-butyl-6-(2-chlorophenyl)-[1,2,4]triazolo[1,5-a]pyrimidine, 5-chloro-7-butyl-6-(2-fluorophenyl)-[1,2,4]triazolo[1,5-a]pyrimidine,
5-chloro-7-butyl-6-(2,6-difluorophenyl)-[1,2,4]triazolo[1,5-a]pyrimidine, 5-chloro-6-(2-chloro-6-fluorophenyl)-7-ethyl-[1,2,4]triazolo[1,5-a]pyrimidine, 5-chloro-6-(2-chloro-6-fluorophenyl)-7-(2-methylpropyl [1 ,2,4]triazolo[1 ,5-a]pyrimidine, 5-chloro-6-(2-chloro-6-fluorophenyl)-7-(2-methyl propyl)-[1 ,2,4]triazolo[1 ,5-a]pyrimidine,
5-chloro-6-(2-chloro-6-fluorophenyl)-7-pentyl-[1,2,4]triazolo[1, 5-a]pyrimidine,
5-chloro-6-(2-chloro-6-fluorophenyl)-7-isopropyl-[1,2,4]triazolo[1,5-a]pyrimidine,
5-chloro-6-(2-chloro-6-fluorophenyl)-7-(1-methylpropyl)-[1,2,4]triazoio[1,5-a]pyrimidine, 5-chloro-6-(2-chloro-6-fluorophenyl)-7-cyclopentyl-[1,2,4]triazolo[1,5-a]pyrimidine, 5-chloro-6-(2-ch loro-6-fluorophenyl)-7-cyclohexyl-[1,2,4]triazolo[1,5-a]pyrimidine, 5-chloro-7-cyclohexyl-6-(2,4,6-trifluorophenyl)-[1,2,4]triazolo[1,5-a]pyrimidine,
7 cyclohexyl-5-methoxy-6-(2,4,6-trifluorophenyl)-[1,2,4]triazolo[1,5-a]pyrimidine,
7-cyclohexyl-6-(2,6-difluorophenyl)-5-methoxy-[1,2,4]triazolo[1,5-a]pyrimidine,
7-cyclohexyl-6-(2-fluorophenyl)-5-methoxy-[1,2,4]triazolo[1,5-a]pyrimidine,
6-(2-chloro-6-fluorophenyl)-7-cyclohexyl-5-methoxy-[1,2,4]triazolo[1,5-a]pyrimidine,
5-chloro-6-(2-chloro-6-fluorophenyl)-7-(4-methylcyclohexyl)[1,2,4]triazolo[1,5-a]pyrimidine, 7-cyclohexyl-5-iodo-6-(2,4,6-trifluorophenyl)-[1,2,4]triazolo[1,5-a]-pyrimidine,
5-chloro-7-cyclohexyl-6-(2,4-difluoro-6-methoxyphenyl)-[1,2,4]triazolo-[1,5-a]pyrimidine, 7-(4-chloro-3-hydroxycyclohexyl)-5-chloro-6-(2,4,6-trifluorophenyl)[1,2,4]triazolo[1, 5-a]pyrimidine, 5-chloro-7-cyclohexyl-6-(2,6-difluoro-4-methoxy-phenyl)-[1,2,4]triazolo[1,5-a]pyrimidine, 5-chloro-7-(cis-4-fluoro-3-cyclohexyl)-6-(2,4,6-trifluorophenyl)[1,2,4]triazolo[1,5-a]pyrimidine, 5-chloro-7-(cis-3-fluoro-3-cyclohexyl)-6-(2,4,6-trifluorophenyl)-[1,2,4]triazolo[1,5-a]pyrimidine, 5-chloro-7-(trans-4-fluoro-3-cyclohexyl)-6-(2,4,6-trifluorophenyl)[1,2,4]triazolo[1,5-a]pyrimidine, 7-cyclohexyl-5-(N-methylamino)-6-(2,4,6-trifluorophenyl)-[1,2,4]triazolo[1,5-a]-pyrimidine, 7-cyclohexyl-5-(N,N-dimethylamino)-6-(2,4,6-trifluorophenyl)[1,2,4]-triazolo[1,5-a]pyrimidine,
5-chloro-7-cyclohex-3-enyl-6-(2,6-difluoro-4-methoxyphenyl)-[1,2,4]triazolo[1,5-a]-pyrimidine, 7-(trans-4-fluoro-3-cyclohexyl)-5-methoxy-6-(2,4,6-trifluorophenyl)-[1,2,4]triazolo[1,5-a]pyrimidme, 7-cyclohexyl-6-(2,6-difluoro-4-methoxyphenyl)-5-ethoxy-[1,2,4]triazolo[1,5-a]pyrimidine, 7-cyclohexyl-6-(2,6-difluoro-4-methoxyphenyl)-5-isopropoxy[1,2,4]triazolo[1,5-a]pyrimidine, 7-cyclohexyl-6-(2,6-difluoro-4-methoxyphenyl)-5-(2,2,2-trifluoroethoxy)[1,2,4]triazolo[1,5-a]pyrimidine, 7-cyclohexyl-6-(2,6-difluoro-4-methoxyphenyl)-5-phenoxy[1,2,4]triazolo[1,5-a]pyrimidine, 7-cyclohexyl-5-benzyloxy-6-(2,6-difluoro-4-methoxyphenyl)[1,2,4]triazolo[1,5-a]pyrimidine, 5-chloro-7-(N-methyl-2,3-dehydroplperid-3-yl)-6-(2,4,6-trlfluorophenyl)-[1,2,4]triazolo[1,5-a]pyrimidine, 5-chloro-6-(2,6-difluorophenyl)-7-(N-methyl-2,3-dehydropiperid-3-yl)[1,2,4]triazolo[1,5-a]pyrimidine,
5-chloro-6-(2,6-difluoro-4-methoxyphenyl)-7-(N-methyl-2,3-dehydropiperid-3-yl)-[1,2,4]triazolo[1, 5-a]pyrimidine, 5-chloro-6-(2-chloro-6-fluorophenyl)-7-(N-methyl-2,3- 3-dehydropiperid-3-yl)[1,2,4]triazolo[1,5-a]pyrimidine, 7-(4-acetoxycyclohexyl)-5-chloro-6-(2,4,6-trifluorophenyl)[1,2,4]triazolo[1,5-a]pyrimidine, 7-(4-acetoxycyclohexyl)-5-chloro-6-(2,6-difluoro-4-methoxyphenyl)[1,2,4]triazolo[1,5-a]pyrimidine, 5-chloro-6-(2,6-difluoro-4-methoxyphenyl)-7-(cis-4-fluorocyclohexyl)[1,2,4]triazolo[1,5-a]-pyrimidine, 5-chloro-6-(2,6-difluoro-4-methoxyphenyl)-7-(trans-4-fluorocyclohexyl)-[1,2,4]triazolo[1, 5-a]pyrimidine, 5-chloro-6-(2,6-difluoro-4-methoxyphenyl)-7-(cis-3-fluorocyclohexyl)[1,2,4]triazolo[1,5-a]pyrimidine, 6-(2,6-difluoro-4-methoxyphenyl)-7-(cis-4-fluorocyclohexyl)-5-methoxy[1,2,4]triazolo[1,5-a]pyrimidine, 6-(2,6-difluoro-4-methoxyphenyl)-7-(trans-4-fluorocyclohexyl)-5-methoxy[1 ,2,4]triazolo[1 ,5-a]-pyrimidine, 6-(2,6-difluoro-4-methoxyphenyl)-7-(cis-3-fluorocyclohexyl)-5-methoxy[1,2,4]triazolo[1,5-a]pyrimidine, 7-cyclohexyl-5-fluoromethoxy-6-(2,4,6-trifluorophenyl)-[1,2,4]triazolo[1,5-a]pyrimidine.

The present invention provides a process for the preparation of a compound of formula I which comprises reacting the corresponding alkyl 2-aryl-3-alkyl-3-oxopropionates of formula IV 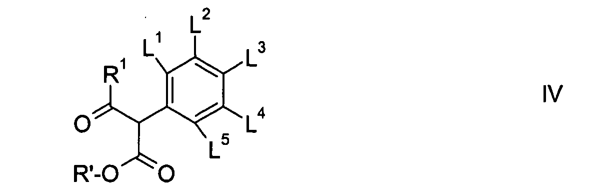 wherein R¹ and L¹, L², L³, L⁴, and L⁵ are as hereinbefore defined for formula I and R' represents an optionally substituted alkyl group,

a) with 2-amino-[1,3,4]-triazole.

This reaction is preferably carried out either at elevated temperatures in the presence of a tertiary amine, in particular tri-n-butylamine, analogously to the methods disclosed in EP 0 770 615, or in the presence of acetic acid analogously to the methods disclosed by G. Fischer in Advances in Heterocyclic Chemistry, Vol. 57, 1993, pages 81-138.

The resulting 7-substituted 5-hydroxytriazolopyrimidine of formula I, wherein X represents a hydroxy group, is subsequently treated with a halogenating agent, preferably selected from the group consisting of phosphorous oxychloride, phosphorous oxybromide, phosphorous pentachloride, phosphorous pentabromide, analogously to the methods disclosed in EP 0 770 615, and

(b) optionally treating the resulting 5-halogen-triazolopyrimidine wich an alcohol or a thioalcohol in the presence of a base, or with a metal amide, a metal alkylamide or a metal dialkylamide, or a metal cyanide.

The compounds of formula I, wherein R¹ represents a fluorocycloalkyl group, can be prepared by reaction of the corresponding compounds of formula I, wherein R¹ represents a cycloalkenyl group, with a fluorination agent, in particular with hydrogenfluoride. The reaction between the 7-cycloalkenyl-triazolopyrimidines of formula I, and hydrogenfluoride is conveniently carried out in the presence of a tertiary amine. Suitable tertiary amines include pyridine, triethylamine, tri-n-butylamine or mixtures of these amines. The reaction is suitably carried out at a temperature in the range from about -20 °C to about +80 °C, the preferred reaction temperature being from about 0 °C to about +40 °C, and most preferably at ambient temperature.

The following examples further illustrate the present invention

### Example 1

### Preparation of 5-chloro-7-n-hexyl-6-(2-chloro-6-fluorophenyl)-1,2,4triazolo[1,5a]pyrimidine

Copper iodide (0.96 g, 5 mmol) is suspended in tetrohydrofuran (THF, 25 ml) under an inert gas atmosphere. The suspension is cooled to about -70 °C and n-hexyllithium (5 ml, 2M in hexanes) is added by syringe. The mixture is stirred for 45 minutes and 5,7-dichloro-6-(2-chloro-6-fluorophenyl)-[1,2,4]triazolo[1,5a]pyrimidine (1.6 g, 5 mmol, obtained according to EP 0 770 615) is added as a solution in THF (10 ml). The reaction mixture is stirred for 15 minutes at about -70°C. The reaction mixture is then quenched with a mixture of aqueous saturated ammonium chloride/concentrated ammonia (9 :1). The two Phase mixture is separated. A brown oil is isolated from the organic layer which is subjected to a chromatographic purification (light petroleum, 20% to 40% ethylacetate), which yields the product as a crystalline residue (0.75 g, m.p. 55-57°C).

### Example 2

### 2A Preparation of ethyl 2-(2-chloro-6-fluorophenyl)-3-(4-methylcyclohexyl)-3-oxopropionate

Lithium diisopropylamide (0.18 mol) in tetrahydrofuran (270 ml) is added to a mixture of ethyl (2-chloro-6-fluorophenyl)acetate (38.1 g, 0.175 mol) and THF (200 ml) at -70°C. The reaction mixture is stirred for 2 hours at about -70°C. 4-Methylcyclohexanecarboxylic acid chloride (28.25 g, 0.175 mol) is added and the reaction mixture is allowed to warm up to room temperature over night. The reaction mixture is then quenched with hydrochloric acid (5N, 60 ml) and most of the organic solvent is distilled off under reduced pressure. From the remainder the product is extracted with light petroleum (200 ml). The organic layer is separated, washed with water, dried with magnesium sulphate and concentrated in vacuo to yield a yellow oil (63.5 g). This is filtered through silica (light petroleum, 3% ethyl acetate) to yield a pale yellow oil (27.2 g). The product is used in the next step without further purification.

### 2B Preparation of 5-hydroxy-7-(4-methylcyclohexyl )-6-( 2-chloro-6-fluorophenyl)-1,2,4-triazolo[1,5a]pyrimidine

A mixture of 2A (3.41 g, 10 mmol ), aminotriazole (0.84 g, 10 mmol) and tributylamine (1.85 g) is heated to 160°C for 2.5 hours. The reaction mixture is cooled and dissolved in water. The mixture is acidified with hydrochloric acid and extracted with ethyl acetate. Drying and evaporation of the organic phase yields a solid which is treated with light petroleum. 1.66 g of a tan powder is obtained (m.p. 235-240°C).

### 2C Preparation of 5-chloro-7-(4-methylcyclohexyl )-6-( 2-chloro-6-fluorophenyl)-[1,2,4]triazolo[1,5a]pyrimidine

A mixture of 2B (1.0 g, 2.77 mmol) and phosphorus oxychloride (2 ml) is heated to 110°C for 3 hours. After cooling the mixture is dissolved in methylene chloride and water is added. The two phase mixture is stirred vigorously for 1 hr. The organic layer is separated, dried and evaporated in vacuo to yield a foam (0.8 g). Upon treatment with diisopropyl ether a tan powder (0.5 g) is obtained which melts at 190-194°C.

### Example 3

### Preparation of 5-methoxy-7-cyclohexyl -6-(2-chloro-6-fluorophenyl)-[1,2,4]triazolo [1,5a]pyrimidine

A mixture of 5-chloro-7-cyclohexyl-6-(2-chloro-6-fluorophenyl )-[1,2,4]triazolo[1,5a]pyrimidine (1.15 g, 3.2 mmol), sodium methylate (0.074 g, 3.2 mmol) and methanol (50 ml) is stirred at ambient temperature for 5.5 hours. The mixture is then poured into water and the product is extracted with methylene chloride. Drying and evaporating the solvent yields a crystalline residue which is treated with a mixture of diisopropylether/light petroleum. 0.85 g of colourless crystals are obtained melting at 193-196°C.

By similar procedures other nucleophilic groups such as azide, cyanide, fluorine, alkylamino, alkylthio, etc. can be introduced.

### Example 4

### Preparation of 5-chloro-7-hydroxymethyl -6-(2-chloro-6-fluorophenyl )-[1,2,4]triazolo[1,5a]pyrimidine

A mixture of 5,7-dichloro-6-( 2-chloro-6-fluorophenyl )- [1,2,4]triazolo[1,5a]pyrimidine (1.9 g, 6 mmol), dibenzoylperoxide (1.04 g, 3 mmol) and molecular sieves 3 A in methanol 50 ml are heated to reflux over night. The mixture is filtered and the sieves are washed thoroughly with ethyl acetate. The combined organic phases are washed with aqueous sodium carbonate, dried and concentrated in vacuo. Upon standing the product starts to crystallize. It is filtered off and washed with toluene and dried in vacuo. Yield: 1.07 g, F.p.: 172-173°C.

The hydroxy group can be derivatized by standard chemistry, e.g. chlorination, alkylation, acetylation etc. to furnish the derivatives listed in the table.

### Example 5

### Preparation of 5-chloro-7-(4-fluorocyclohexyl )-6-(2,4,6-trifluorophenyl)-[1,2,4]triazolo[1,5a]pyrimidine

A mixture of 5-chloro-7-(4-cyclohex-3-enyl)-6-(2,4,6-trifluorophenyl)-[1,2,4]triazolo[1,5a]pyrimidine (1.3 g, 3.5 mmol) and hydrogenfluoride in pyridine (70%, 8 ml) is stirred at ambient temperature for 2 hours. The mixture is then poured onto a mixture of ice/sodium hydrogencarbonate. The product is extracted from this mixture with ethyl acetate. Drying of the organic phase with magnesium sulfate and evaporation yields 1.4 g of a colourless oil. This is purified by flash chromatograpy giving rise to two product fractions: A, 0.35 g a colourless solid (m.p.: 153°C) which is a mixture (1 : 2) of the trans 4-F and the trans 3-F products and B, 0.82 g a colourless solid (m.p.: 162-166°C) being a mixture (6 : 1) of the 4-cis-F and 3 trans-F products as indicated by NMR analysis.

### Example 6

### Preparation of 5-chloro-7-(N-methyl-2,3-dehydropiperid-3-yl)-6-(2,4,6-trifluorophenyl)-[1,2,4]triazolo[1,5a]pyrimidine

To a solution of 5,7-dichloro-6-(2,4,6-trifluorophenyl)-1,2,4-triazoio[1,5a]pyrimidine (1.0 g, 3.1 mmol) in methylene chloride (10 ml) is added N-methyl-2,3-dehydropiperidine (10 mmol) and triethylamine (0.5 ml). The mixture is stirred over night. The reaction mixture is extracted with aqueous 1 N hydrogen chloride, water and brine. It is dried and evaporated in vacuo. The crude product is purified by flash chromatography using light petroleum/ethyl acetate (1 :1) as the eluent. Evaporation of the product containing fractions gives 0.55 g of bright orange crystals melting at 175°C.

### Example 7

### Synthesis of 5-methoxy-6-aryl-7-alkyl-[1,2,4]triazolo[1,5a]pyrimidines

### 7A Prepäration of 5-chloro-7-cyclohexyl-6-(2,6-difluorophenyl)- [1,2,4]triazolo[1,5a]pyrimidine

To a solution of zinc bromide (8.1 g, 36 mmol) in 50 ml dry THF is added cyclohexylmagnesium chloride (2M in ether, 18 ml, 36 mmol). The milky white suspension is stirred at ambient temperature for 1 h. In a separate flask lithium chloride (3.05 g, 72 mmol) is dried at about 130°C at 0.1 mbar for 1 h. CuCN (3.22 g, 36 mmol) is added and the flask is purged with argon. THF (36 ml) is added and the clear pale green solution is transferred to the previously prepared suspension of the cyclohexylzinc, cooled to -10°C, by syringe. The mixture is stirred at 0°C for 10'. It is then cooled to - 25°C and 5,7-dichloro-6-(2,6-difluorophenyl)-[1,2,4]triazoio[1,5a]pyrimidine (9.05 g, 30 mmol) is added as a solution in 30 ml THF. The mixture is allowed to warm to ambient temperature. Stirring is continued over night. The reaction mixture is then quenched with 100 ml of a mixture of aq. saturated ammonium chloride/conc. ammonia (9:1) and the two phase mixture is separated. The aqueous phase is extracted with dichloromethane. The organic phases are combined, dried and concentrated in vacuo. The resulting residue is treated with light petroleum. The tan crystals are recrystalized from isopropanol to yield colorless crystals, 7.11 g, m.p. 180-184°C.

### 7B Preparation of 5-methoxy-7-cyclohexyl-6-(2,6-difluorophenyl)-[1,2,4]triazolo[1,5a]pyrimidine

To a solution of 7A (0.25 g, 0.7 mmol) in 10 ml dry methanol is added methanolic sodium methoxide (1.4 ml, 0.7 mmol). The reaction mixture is stirred at ambient temperature for 1 hour. It is then quenched with water and the product is extracted with dichloromethane. Drying and evaporating the organic phases yields a colorless crystalline residue (0.22 g, 92%, m.p. 190-196°C) which does not require further purification.

### Examples 8-100

Using the synthetic procedures described in Examples 1 to 7, the following compounds are prepared and their structure and melting point are given in Table 1 below.

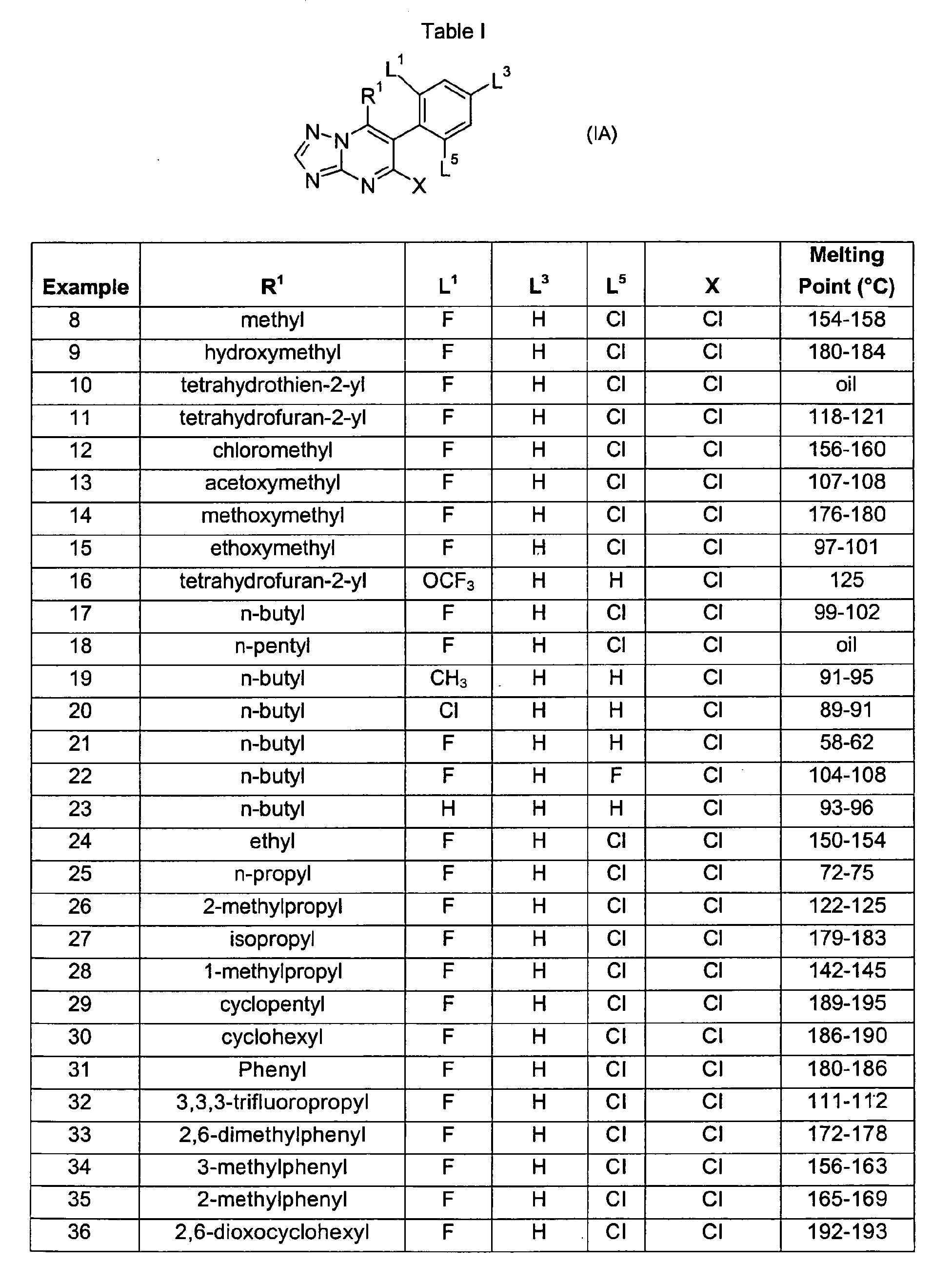

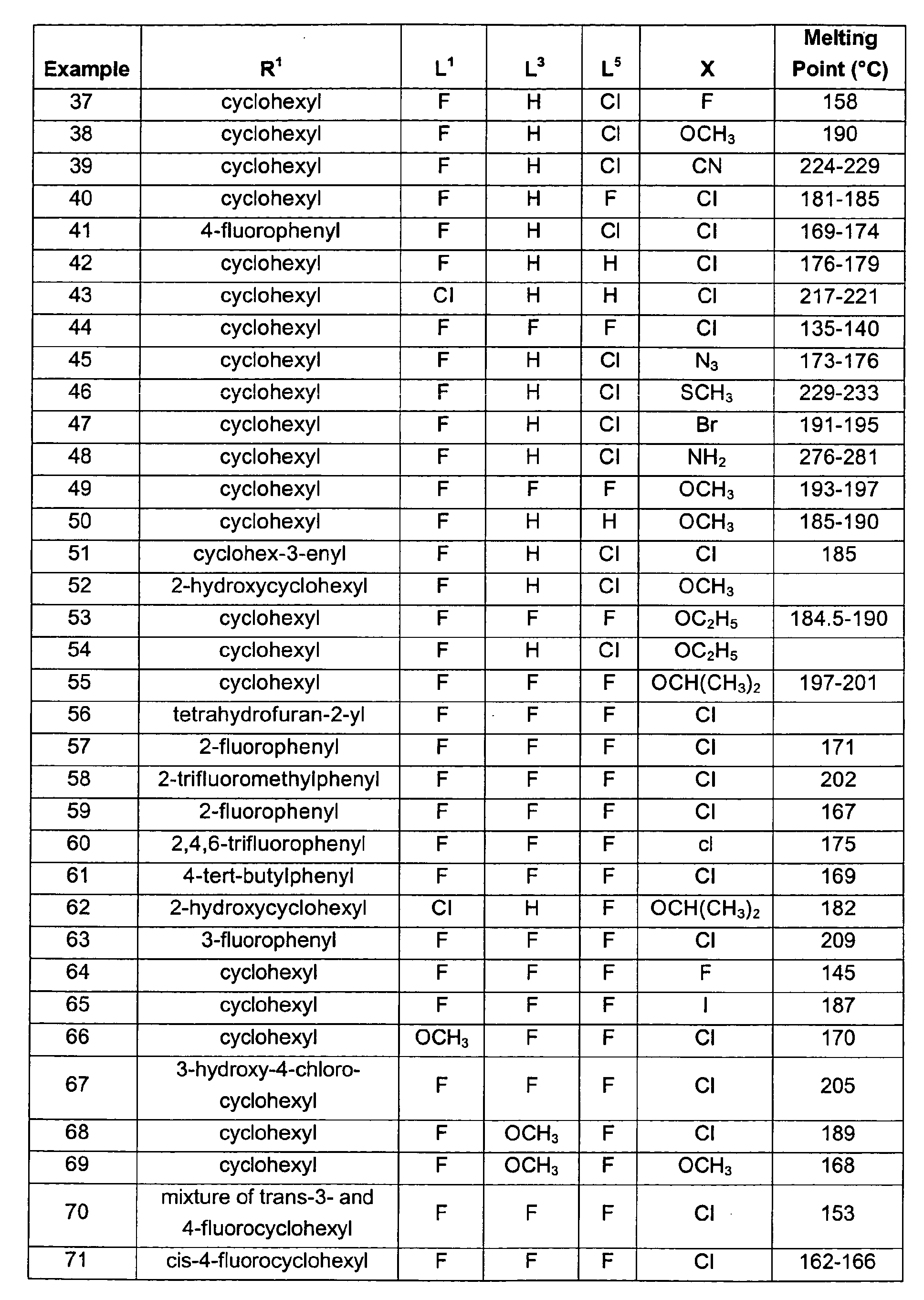

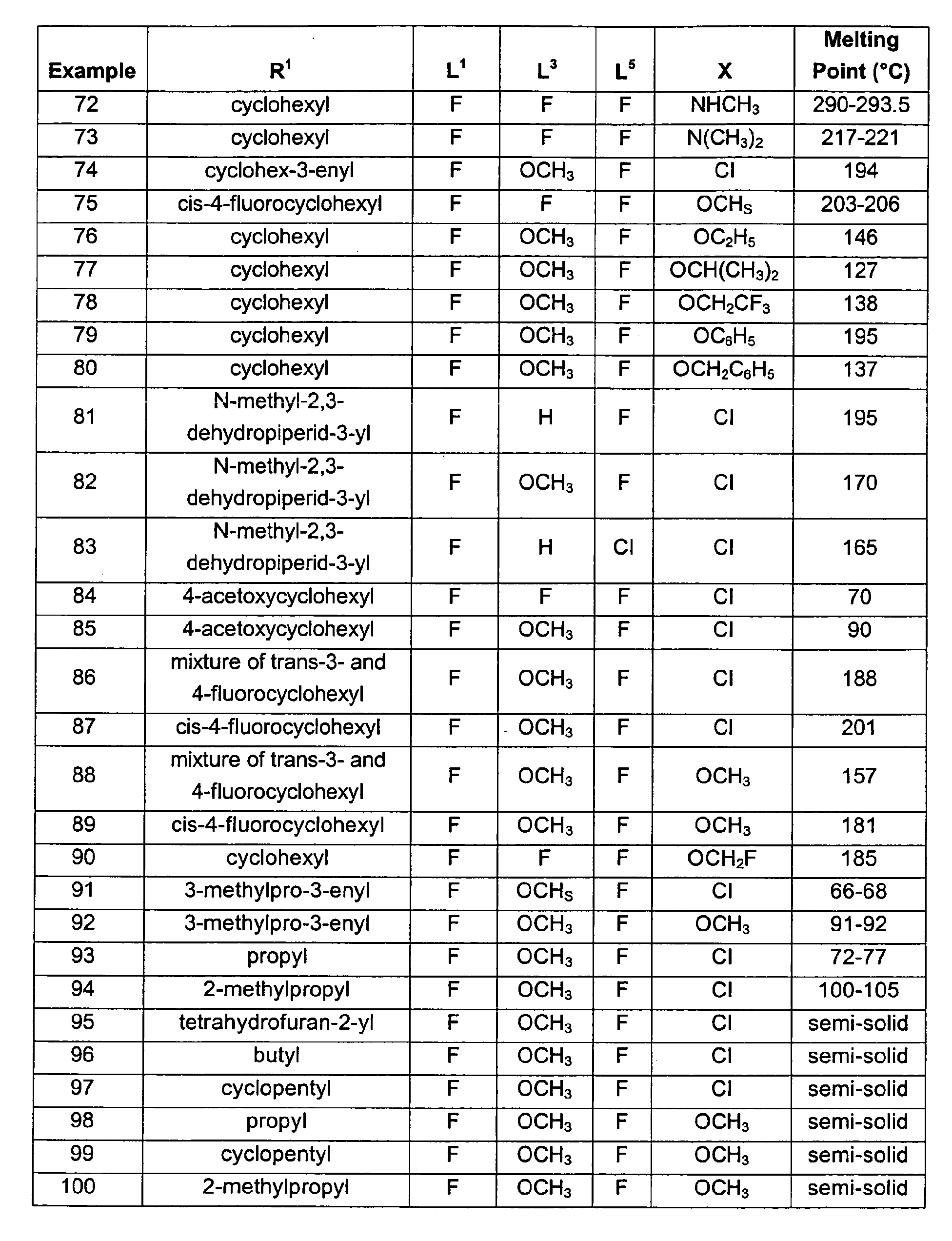

## Claims

1. A process for the preparation of compounds of formula I 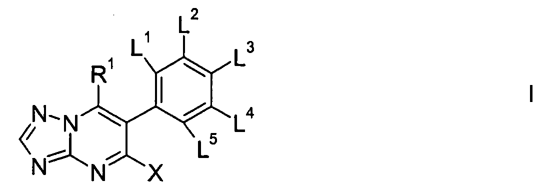 wherein
R¹ represents an unsubstituted or substituted straight or branched shain alkyl, alkynyl, alkadienyl, having up to 10 carbon atoms, or aryl group having 6, 10 or 14 carbon atoms or an unsubstituted or substituted C₃-C₈-cycloalkyl or C₅-C₈-cycloalkenyl group, a substituted or unsubstituted saturated or unsaturated heterocyclyl group having 5 or 6 ring atoms selected from carbon, oxygen, sulfur and nitrogen, one of which being oxygen, sulfur or nitrogen;
X represents a hydrogen or halogen atom, or a hydroxy, alkoxy, aryloxy, aralkyloxy, haloalkoxy, alkylthio, cyano, amino, alkylamino or dialkylamino group; wherein alkyl moieties have up to 10 carbon atoms and aryl groups have 6, 10 or 14 carbon atoms;
L¹, L², L³, L⁴ and L⁵ each independently represent hydrogen, halogen, nitro or cyano or an unsubstituted or substituted C₁-C₁₀-alkyl or C₁-C₁₀-alkoxy group, the groups being unsubstituted or substituted by one or more halogen atoms, nitro, cyano, C₁-C₆-alkyl or C₁-C₆-alkoxy,
which comprises reacting alkyl 2-aryl-3-alkyl-3-oxopropionates of formula IV 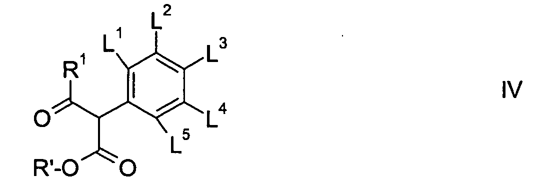 wherein R¹ and L¹, L², L³, L⁴ and L⁵ are as defined for formula I and R' represents an optionally substituted alkyl group,
a) with 2-amino-[1,3,4]-triazole, and treating the resulting 5-hydroxytriazolopyrimidines of formula I, wherein X represents a hydroxy group, with a halogenating agent, and
b) optionally treating the resulting 5-hydroxytriazolopyrimidines with an alcohol or a thioalkohol in the presence of a base, or with a metal amide, a metal alkyl amide or a metal dialkylamide, or a metal cyanide.

2. A process according to claim 1, wherein step a) is carried out at elevated temperatures in the presence of a tertiary amine.

3. A process according to claim 1, wherein step a) is carried out in the presence of acetic acid.

4. Intermediates of formula IV as defined in claim 1.

5. Intermediates of formula IV according to claim 4, wherein the phenyl group 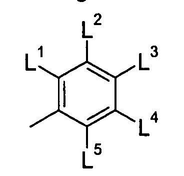 is selected from 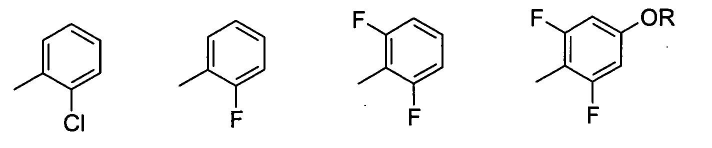 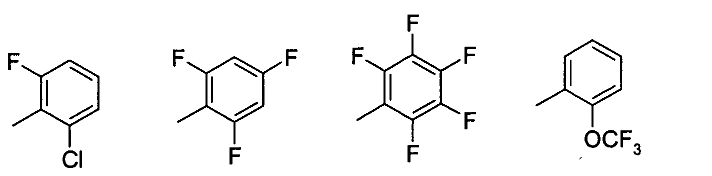 wherein R represents a C¹-C⁶- alkyl group.
